# EUROPÄISCHE PATENTSCHRIFT

(11) **EP 2 504 698 B1**
(45) Veröffentlichungstag und Bekanntmachung des Hinweises auf die Patenterteilung: **14.03.2018**
(21) Anmeldenummer: 10787078.4
(22) Anmeldetag: 25.11.2010
(51) Int. Cl.: G01N 33/50

(54) **VERFAHREN ZUM VORHERSAGEN DES ANSPRECHENS EINER TUMORERKRANKUNG AUF EINE THERAPEUTISCHE MASSNAHME**
METHOD FOR PREDICTING THE RESPONSE OF A TUMOUR DISEASE TO A THERAPEUTIC MEASURE
PROCÉDÉ PERMETTANT DE PRÉVOIR LA RÉPONSE D'UNE PATHOLOGIE TUMORALE À UNE MESURE THÉRAPEUTIQUE

(30) Priorität: 25.11.2009 DE 102009047146
(43) Veröffentlichungstag der Anmeldung: 03.10.2012
(73) Patentinhaber: Pachmann, Ulrich, 95448 Bayreuth (DE); Pachmann, Katharina, 95448 Bayreuth (DE)
(72) Erfinder: Pachmann, Ulrich, 95448 Bayreuth (DE); Pachmann, Katharina, 95448 Bayreuth (DE)
(74) Vertreter: Dr. Gassner & Partner mbB
(86) Internationale Anmeldenummer: PCT/EP2010/068235
(87) Internationale Veröffentlichungsnummer: WO 2011/064308

(56) Entgegenhaltungen:
- WO-A1-03/065042
- DE-A1- 19 833 738
- US-A1- 2007 071 762
- PACHMANN K ET AL: "Chemosensitivity Testing of Circulating Epithelial Cells (CETC) in Breast Cancer Patients and Correlation to Clinical Outcome" CANCER RESEARCH, Bd. 69, Nr. 24, Suppl. 3, 13. Dezember 2009 (2009-12-13), Seite 606S, XP009143042 AMERICAN ASSOCIATION FOR CANCER REREARCH, US ISSN: 0008-5472
- BOSANQUET A G ET AL: "AN ASSESSMENT OF A SHORT-TERM TUMOR CHEMO SENSITIVITY ASSAY IN CHRONIC LYMPHOCYTIC LEUKEMIA" BRITISH JOURNAL OF CANCER, Bd. 47, Nr. 6, 1983, Seiten 781-789, XP002616234 ISSN: 0007-0920

## Beschreibung

Die Erfindung betrifft ein Verfahren zum Vorhersagen des Ansprechens einer von einem soliden epithelialen Tumor verursachten Tumorerkrankung eines Patienten auf eine therapeutische Maßnahme. Unter einem soliden Tumor wird ein Tumor verstanden, dessen Zellen im Zellverbund wachsen und dabei ein festes, örtlich umschriebenes Gewebe bilden. Es ist bekannt, dass sich Zellen aus einem solchen Tumor ablösen und über Blut oder Lymphe im Körper verteilt werden.

Es ist weiterhin bekannt, dass Tumore unterschiedlich sensitiv gegenüber therapeutischen Maßnahmen, wie beispielsweise einer Chemotherapie oder einer Bestrahlung, sind. Die Empfindlichkeit von Krebszellen gegenüber im Rahmen einer Chemotherapie verabreichten wachstumshemmenden Chemotherapeutika, den so genannten Zytostatika, wird als Chemosensitivität bezeichnet. Der Erfolg einer Chemotherapie hängt unter anderem von der Chemosensitivität der Krebszellen ab. Beispielsweise kann die Chemosensitivität gegenüber Zytostatika aus der Gruppe der Alkylantien, die über eine Erbgutveränderung der Tumorzellen wirken, durch die Aktivierung von DNA-Reparaturenzymen in Tumorzellen vermindert sein. Ebenso kann ein verminderter Transport von Zytostatika in das Zellinnere, die Inaktivierung der Zytostatika oder auch die fehlende Expression von wirkungsvermittelnden Rezeptoren zu einer Verringerung der Chemosensitivität der Tumorzellen führen. Eine verringerte Chemosensitivität der Tumorzellen kann ein Ausbleiben des Therapieerfolgs bewirken.

Es wird angenommen, dass jeder Patient wegen seines genetischen Hintergrunds und der Entwicklung von Subklonen innerhalb eines Tumors individuell auf ein bestimmtes Chemotherapeutikum reagiert. Um für einen Patienten das für ihn effektivste Chemotherapeutikum zu ermitteln, werden zurzeit vor Behandlung des Tumors zunächst Zellen aus Gewebestücken des Tumors gewonnen, indem das Tumorgewebe der Gewebestücke mit proteolytisch wirksamen Enzymen verdaut wird. Anschließend wird die Chemosensitivität der aus dem Tumorgewebe gewonnenen und über längere Zeit kultivierten Zellen gegenüber verschiedenen Chemotherapeutika in-vitro ermittelt. Dazu wird geprüft, ob sich die kultivierten Zellen in Gegenwart eines bestimmten Chemotherapeutikums vermehren oder ob sie in dessen Gegenwart absterben. Durch das Verfahren soll weitgehend verhindert werden, dass ein Patient mit einem für ihn unwirksamen oder schlecht wirksamen Chemotherapeutikum behandelt wird.

Aus Bird, M. C. et al., Leuk. Res. 1986, 10(4); 445-449 ist ein in-vitro Chemosensitivitätstest für hämatologische Tumore bekannt. Dabei werden Leukozyten aus Blut oder Knochenmark isoliert, einem Therapeutikum ausgesetzt und für 4 Tage inkubiert. Die Abtötung von Tumorzellen durch das Therapeutikum wurde durch differenzierte Färbung von lebenden und toten Zellen bestimmt, wobei die lebenden Zellen morphologisch identifiziert wurden. Nachteilig ist bei diesem Verfahren, dass es sich nur zum Testen der Chemosensitivität hämatologischer Tumore eignet.

Aus Pachmann, K. et al., J. Clin. Oncol. 2008, 26:1208-1215 ist es bekannt, dass das Rückfallrisiko von Brustkrebspatientinnen, welche eine adjuvante Chemotherapie erhalten, durch Bestimmen der Anzahl von im Blut zirkulierenden epithelialen Tumorzellen (CETCs) während und am Ende der Chemotherapie mit einer gewissen Wahrscheinlichkeit vorhergesagt werden kann. Es wird vermutet, dass die Zunahme der CETCs von bereits wachsenden Metastasen, die Zellen in den Kreislauf abstoßen, ausgeht. Das Überwachen der Anzahl der CETCs wird als wertvolles Werkzeug für die Therapieüberwachung erachtet.

Aus Veneziani, B. M. et al., Mol. Cancer Ther. 2007, 6(12), Seiten 3091 bis 3099 ist es bekannt, aus soliden epithelialen Tumoren Gewebe zu entnehmen und daraus Stromazellen und epitheliale Zellen zu isolieren und diese Zellen in Kokultur für mindestens 15 Tage zu kultivieren. Nach Passagieren der Zellen wurden diese verschiedenen Therapeutika ausgesetzt und die Hemmung des Zellwachstums der Tumorzellen untersucht, um Hinweise auf die Effizienz der Therapeutika zu erhalten. Nachteilig bei diesem Verfahren ist, dass dessen Ausführung verhältnismäßig lange dauert.

Aus der US 2007/071762 A1 ist es bekannt, zur Selektion eines chemotherapeutischen Agens zur Behandlung von Krebs zirkulierende Krebszellen aus Blut anzureichern und die Expression einer Reihe von Medikamentenansprechindikatoren unter Verwendung von Antikörpern in mindestens einer Zelle der Tumorzellprobe zu bestimmen und auf der Basis dieser Expression ein chemotherapeutisches Agens auszuwählen. Bei den Medikamentenansprechindikatoren kann es sich um zelluläre Komponenten handeln, welche mit dem Wirkmechanismus des chemotherapeutischen Agens zusammenhängen. Beispielsweise kann es sich dabei um das DNA-Reparaturprotein ERCC1, Beta-Tubulin III, Thymidylat-Synthase, Topoisomerase II, Topoisomerase I oder Ribonuklease-Reduktase handeln. Gemäß Beispiel 7 wird ein in vitro System beschrieben, das das zytotoxische Ansprechen kultivierter humaner Zelllinien auf Antikrebsmedikamente beschreibt. Um das Ansprechen von Antikrebsmedikamenten vorherzusagen, werden Zelllinien verwendet. Die Zellen werden dazu ausgesät und 24 Stunden bei 37°C in 5% CO₂-Atmosphäre gehalten. Danach werden die Zellen dem jeweiligen Medikament ausgesetzt. Im Falle von Tamoxifen werden die Zellen 72 Stunden bis zur Konfluenz kultiviert. Anschließend wird Medium mit 0,5% FCS (fötales Kälberserum) zugesetzt, um die Östrogenrezeptorexpression zu maximieren. Kontrollen enthalten ein entsprechendes Medium und werden gleich behandelt wie die behandelten Zellen. 72 Stunden nach Behandlung wird mittels eines Tetrazolium-Salzes die Anzahl lebender Zellen für jede Medikamentenkonzentration bestimmt. Als Ergebnis wird die prozentuale Hemmung des Wachstums im Vergleich zur Medikamentenkonzentration erhalten.

Die DE 198 33 738 A1 offenbart ein Verfahren zur Isolierung von Krebszellen aus Körperflüssigkeiten, bei dem die Krebszellen durch ein Sieb zurückgehalten werden. Die isolierten Zellen können in vitro unter Zusatz von Zytostatika kultiviert werden, so dass verschiedene Therapieformen in vitro getestet und für jeden Patienten individuell optimiert werden können.

Die WO 03/065042 A1 betrifft ein Verfahren zum Beurteilen des Vorhandenseins einer Malignität, bei dem eine Probe mit einer gemischten Zellpopulation, die hämatopoetische und nichthämatopoetische maligne Zellen enthält, mit einem nachweisbaren spezifisch mit den malignen Zellen reagierenden Liganden und mindestens einem die malignen Zellen ebenfalls spezifisch markierenden Reagenz in Kontakt gebracht wird. Anschließend wird die Zahl der markierten Zellen analysiert, um das Vorhandensein und die Anzahl maligner Zellen zu bestimmen. Weiterhin werden Veränderungen in mindestens einem Tumordiathese-assoziierten Molekül bestimmt. Die markierten malignen Zellen können mit einem chemotherapeutischen Agens in Kontakt gebracht werden, um deren Sensitivität demgegenüber zu bestimmen.

Aus Bosanquet, A. G. et al., Br. J. Cancer 1983, 47, Seiten 781 bis 789 ist die Ermittlung der Chemosensitivität in Zellen chronischer lymphatischer Leukämien, die im Gegensatz zu Zellen epithelialer Tumorzellen in verhältnismäßig großer Anzahl im Blut betroffener Patienten vorkommen, bekannt. Bei dem Verfahren werden weiße Zellen aus peripherem Blut isoliert, einem Medikament ausgesetzt und für 4 Tage kultiviert. Das durch die Medikamente induzierte Abtöten von Tumorzellen wird durch unterschiedliche Färbungen toter und lebender Zellen bestimmt. Anschließend wird die Viabilität der Tumorzellen berechnet. Gemäß Seite 782, rechte Spalte, vierter Absatz enthält das zur Kultivierung der Zellen eingesetzte RPMI-Medium fötales Kälberserum (FCS).

Aus Weisenthal, L. M. und Lippman, M. E., Cancer Treatment Reports 69, No. 6, 1985, Seiten 615 bis 632 und Weisenthal, L. M. et al., Recent Results in Cancer Research 94, Seiten 161 bis 173 ist es bekannt, aus einer Gewebeprobe eines soliden Tumors eine Zellpopulation zu gewinnen und damit einen Chemosensitivitätstest durchzuführen. Der Test kann als klonogener oder nichtklonogener Test durchgeführt werden.

Aufgabe der vorliegenden Erfindung ist es, ein alternatives schnell durchzuführendes Verfahren anzugeben, welches vor Durchführen einer therapeutischen Maßnahme gegen eine von einem soliden epithelialen Tumor ausgehende Tumorerkrankung eine Vorhersage darüber erlaubt, ob der Tumor gegenüber der therapeutischen Maßnahme sensitiv ist.

Diese Aufgabe wird durch ein Verfahren gemäß Anspruch 1 gelöst. Zweckmäßige Ausgestaltungen ergeben sich aus den Merkmalen der Patentansprüche 2 bis 11.

Erfindungsgemäß ist ein Verfahren zum Vorhersagen des Ansprechens einer von einem soliden epithelialen Tumor verursachten Tumorerkrankung eines Patienten auf eine therapeutische Maßnahme vorgesehen, wobei epitheliale Tumorzellen aus einer Körperflüssigkeit des Patienten jeweils in einem Zellkulturmedium aufgenommen werden. Bei den in der Körperflüssigkeit enthaltenen epithelialen Tumorzellen handelt es sich um vom soliden Tumor abgelöste Zellen.

Das Zellkulturmedium ist ein Medium, das die Lebensfähigkeit der Tumorzellen in Zellkultur erhält. Zahlreiche derartiger Medien, wie z. B. Ham's F12 oder RPMI, sind bekannt. Das Zellkulturmedium enthält keinen zugesetzten Wachstumsfaktor bzw. kein Serum, wie beispielsweise fötales Kälberserum. Dadurch wird eine Selektion der epithelialen Tumorzellen gegenüber anderen eventuell enthaltenen Zellen bewirkt, weil die Tumorzellen im Gegensatz zu den anderen Zellen keine von außen zugesetzten Wachstumsfaktoren zum Überleben bzw. Vermehren benötigen.

Bei dem Verfahren werden die Tumorzellen aus einer Probe des die Tumorzellen enthaltenden Zellkulturmediums der therapeutischen Maßnahme ausgesetzt, während die Tumorzellen aus einer Kontrollprobe des die Tumorzellen enthaltenden Zellkulturmediums unbehandelt bleiben. Anschließend wird für die Probe und die Kontrollprobe jeweils der Anteil absterbender und toter epithelialer Tumorzellen an der Gesamtzahl der epithelialen Tumorzellen und daraus eine durch die therapeutisehe Maßnahme bedingte Absterberate der epithelialen Tumorzellen als Maß für das Ansprechen bestimmt. Der Anteil absterbender und toter epithelialer Tumorzellen kann auch indirekt bestimmt werden, indem der Anteil lebender und nicht absterbender epithelialer Tumorzellen an der Gesamtzahl epithelialer Tumorzellen bestimmt und von dem der Gesamtzahl entsprechenden Ganzen (100 % oder 1) abgezogen wird. Der Anteil absterbender und toter bzw. lebender und nichtabsterbender epithelialer Tumorzellen kann mit Hilfe zytometrischer oder bildanalytischer Verfahren bestimmt werden.

Die durch die therapeutische Maßnahme bedingte Absterberate der epithelialen Tumorzellen wird bestimmt, indem der Anteil absterbender und toter epithelialer Tumorzellen in der Kontrollprobe von dem Anteil absterbender und toter epithelialer Tumorzellen in der Probe abgezogen wird. Die Absterberate kann zeitabhängig und/oder in Abhängigkeit von der Konzentration eines Chemotherapeutikums oder der Intensität einer therapeutischen Maßnahme, bspw. einer Bestrahlung, bestimmt werden.

Ein großer Vorteil im Hinblick auf die Belastung des Patienten und auf eine schnelle Ausführung des Verfahrens besteht darin, dass zur Durchführung des Verfahrens kein aus dem soliden epithelialen Tumor zu entnehmendes Material benötigt wird.

Bei dem erfindungsgemäßen Verfahren ist es weiterhin nicht erforderlich, die epithelialen Tumorzellen zu kultivieren bevor sie der therapeutischen Maßnahme ausgesetzt werden. Die Tumorzellen können direkt nach der Aufnahme in dem Zellkulturmedium der therapeutischen Maßnahme ausgesetzt werden.

Zwischen der therapeutischen Maßnahme und dem Bestimmen des Anteils absterbender und toter epithelialer Tumorzellen an der Gesamtzahl der epithelialen Tumorzellen kann eine Zeit von einer Stunde bis zu einigen Tagen, insbesondere von 2 Stunden, 3 Stunden, 1 Tag, 2 Tage oder 3 Tage, liegen. In dieser Zeit werden die Tumorzellen unter üblichen Zellkulturbedingungen gehalten, welche ohne therapeutische Maßnahme ein Überleben der Tumorzellen im Zellkulturmedium erlauben. Üblicherweise umfassen solche Zellkulturbedingungen einen CO₂-Gehalt der Atmosphäre von 5 % - 10 % und eine Temperatur von 37° C. Das Zellkulturmedium enthält keinen zugesetzten Wachstumsfaktor und kein zugesetztes Serum, welches Wachstumsfaktoren enthält.

Die Erfinder haben erkannt, dass sich die Reaktion von in einer Körperflüssigkeit eines Patienten vorhandenen Zellen eines soliden epithelialen Tumors auf eine therapeutische Maßnahme dazu eignet, das Ansprechen einer von diesem Tumor verursachten Tumorerkrankung auf die therapeutische Maßnahme vorherzusagen. Das ist erstaunlich, weil man bisher angenommen hat, dass die meisten der in der Körperflüssigkeit vorhandenen Tumorzellen deshalb in der Körperflüssigkeit vorhanden sind, weil sie ihre Fähigkeit verloren haben, in Assoziation mit anderen Zellen des Tumors zu wachsen. Man hat daher angenommen, dass sie für einen soliden Tumor nicht repräsentativ seien. Die Erfinder haben aber erkannt, dass sich die in einer Körperflüssigkeit vorhandenen epithelialen Tumorzellen so verändern können, dass sie sich wieder ansiedeln und im Zellverbund wachsen können. Damit spielen sie bei der oft lebensbedrohlichen Metastasenbildung eine entscheidende Rolle.

Bei der Tumorerkrankung kann es sich auch um eine minimale Resterkrankung oder ein Wiederauftreten der Tumorerkrankung in Form von Metastasen nach kompletter Entfernung eines Primärtumors handeln. In der Körperflüssigkeit können epitheliale Tumorzellen oft auch noch Jahre nach der Entfernung eines soliden epithelialen Primärtumors vorhanden sein. In einem solchen Fall wäre eine Gewebeentnahme aus dem soliden Tumor, wie sie bei Veneziani et al. beschrieben ist, gar nicht möglich.

Durch das erfindungsgemäße Verfahren ist es möglich, vor Beginn einer Therapie ohne Entnahme einer Gewebeprobe aus dem soliden Tumor eines Patienten zu prüfen, ob eine ins Auge gefasste therapeutische Maßnahme zur Therapie der Tumorerkrankung geeignet ist. Es erlaubt eine individuelle patientenspezifische Therapie mittels derjenigen therapeutischen Maßnahme, auf welche der jeweilige Patient am besten anspricht. Gleichzeitig kann durch das erfindungsgemäße Verfahren vermieden werden, dass der Patient mit einer für ihn unwirksamen oder schlecht wirksamen und nebenwirkungsreichen teuren Therapie behandelt wird. Abgesehen von den individuellen Vorteilen für den einzelnen Patienten können mittels des erfindungsgemäßen Verfahrens Kosten im Gesundheitswesen eingespart werden.

Das erfindungsgemäße Verfahren kommt ohne für epitheliale Tumorzellen spezifische Anreicherungsverfahren, beispielsweise mittels Antikörper-beschichteten magnetischen Partikeln aus. Dadurch ist eine Verfälschung der ermittelten Absterberate durch für verschiedene Zellen unterschiedliches oder unvollständiges Anreichern ausgeschlossen. Darüber hinaus kommt das erfindungsgemäße Verfahren ohne eine die Eigenschaften der Tumorzellen möglicherweise verändernde Isolierung der Zellen aus dem Tumorgewebe mittels enzymatischem Verdau und ohne eine bei Chemosensitivitätstests bisher übliche Langzeitkultivierung der Tumorzellen aus. Bei der Langzeitkultivierung können sich die Eigenschaften, wie z.B. die Sensitivität der Tumorzellen gegenüber der therapeutischen Maßnahme, verändern. Durch die Isolierung der Zellen und die Langzeitkultivierung kann es daher zu einer Verfälschung der Ergebnisse kommen. Mittels des erfindungsgemäßen Verfahrens kann dagegen die Sensitivität des Tumors gegenüber der therapeutischen Maßnahme spezifisch und selektiv an den aus der Körperflüssigkeit gewonnenen epithelialen Tumorzellen bestimmt werden.

Das erfindungsgemäße Verfahren ist damit deutlich schneller durchzuführen und führt schneller zu einem Ergebnis als das beispielsweise aus Veneziani et al. bekannte Verfahren. Es ist nicht erforderlich, dem Patienten Gewebe des soliden epithelialen Tumors zu entnehmen und die daraus gewonnenen Zellen zu kultivieren, bevor sie der therapeutischen Maßnahme ausgesetzt werden. Nachdem die Zellen der therapeutischen Maßnahme ausgesetzt wurden, ist kein Wachstum der Zellen erforderlich, weil beim erfindungsgemäßen Verfahren keine Wachstumsinhibition zur Beurteilung der Effizienz der therapeutischen Maßnahme bestimmt werden muss.

Bei der Körperflüssigkeit kann es sich um Blut, Aszitesflüssigkeit, Lymphe, Pleuraexsudat, Liquor oder Urin handeln. Die epithelialen Tumorzellen können dabei, insbesondere im Blut vorkommende, zirkulierende epitheliale Tumorzellen (CETCs) sein.

Vor der Aufnahme in das Kulturmedium können die Tumorzellen allein unter Ausnutzung ihres gegenüber der sie umgebenden Körperflüssigkeit höheren spezifischen Gewichts, insbesondere durch Absetzenlassen oder Zentrifugieren, angereichert werden. Wenn es sich bei der Körperflüssigkeit um Blut handelt, können die darin enthaltenen roten Blutkörperchen vor dem Anreichern der Tumorzellen lysiert werden. Dies erleichtert eine spätere optische, insbesondere bildanalytische, Identifizierung der epithelialen Tumorzellen.

Zur Unterscheidung von sonstigen in der Körperflüssigkeit vorhandenen Zellen können die epithelialen Tumorzellen mit einer spezifisch die Tumorzellen markierenden Substanz, insbesondere einem für epitheliale Zellen spezifischen Antikörper, in Kontakt gebracht werden. Bei dem Antikörper kann es sich beispielsweise um einen das humane epitheliale Antigen erkennenden Antikörper, beispielsweise den monokularen Antikörper HEA-125, handeln. Das humane epitheliale Antigen, welches auch als HEA oder als CD326 bezeichnet wird, kommt auf Blutzellen nicht vor. Da epitheliale Zellen üblicherweise nicht in der Körperflüssigkeit vorkommen, reicht ein spezifisch epitheliale Zellen markierender, insbesondere färbender, Antikörper aus, um diese als epitheliale Tumorzellen zu identifizieren. Die spezifisch die Tumorzellen markierende Substanz ist eine Substanz, welche die Lebensfähigkeit der epithelialen Tumorzellen nicht oder zumindest nicht wesentlich beeinflusst.

Zur Identifizierung der absterbenden und toten epithelialen Tumorzellen bzw. der lebenden und nicht absterbenden Tumorzellen kann den epithelialen Tumorzellen ein spezifisch absterbende und/oder tote Zellen anzeigender erster Indikator, insbesondere Propidiumiodid, oder ein spezifisch lebende Zellen anzeigender zweiter Indikator zugesetzt werden. Bei Zusatz von Propidiumiodid bleiben die lebenden Zellen aufgrund ihrer intakten Zellmembran ungefärbt, während die toten und absterbenden Zellen von dem in die Zellen eindringenden Propidiumiodid gefärbt werden.

Die therapeutische Maßnahme kann eine Exposition gegenüber Strahlung oder Wärme oder ein Inkontaktbringen mit einem Zytostatikum, insbesondere 5-Fluoruracil, oder einem sonstigen gegen den Tumor gerichteten Therapeutikum umfassen. Bei dem soliden epithelialen Tumor kann es sich um ein Mammakarzinom oder ein Bronchialkarzinom handeln.

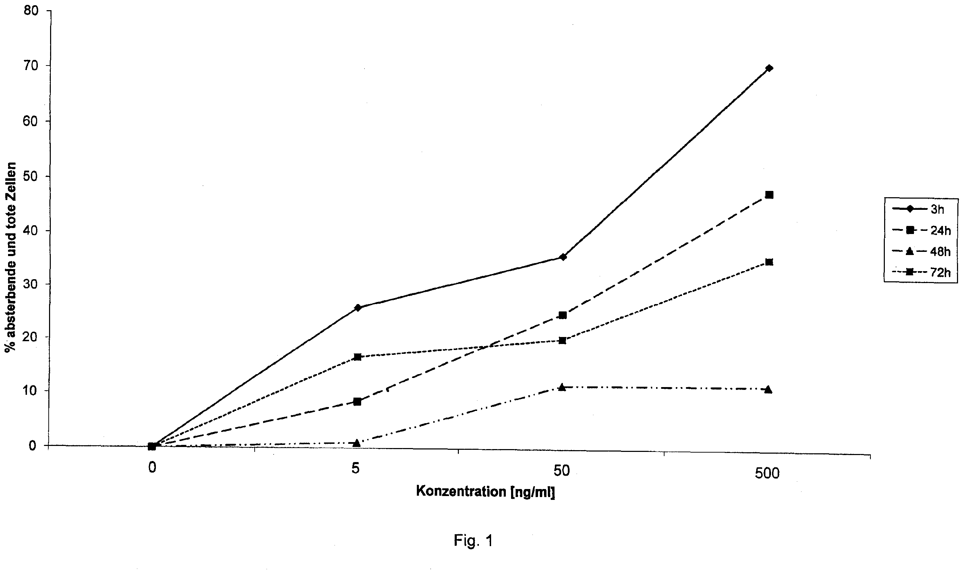

Nachfolgend wird die Erfindung anhand eines Ausführungsbeispiels und der Figur 1 erläutert.

Fig. 1 zeigt die Absterberate von Zellen, welche das humane epitheliale Antigen (HEA) tragen, nach Inkubation mit 5-Fluoruracil und Färbung mit Propidiumiodid in Abhängigkeit der Konzentration des eingesetzten 5-Fluoruracils und der Inkubationszeit.

3 ml mit EDTA als Antikoagulanz versetztes Blut werden mit 30 ml Ammoniumchlorid-Lysereagenz für rote Blutzellen (Qiagen, 40724 Hilden, Deutschland) versetzt und 10 Minuten bei 8 bis 12° C inkubiert. Die Leukozyten und die epithelialen Tumorzellen werden durch Zentrifugation für 7 Minuten bei 700 x g sedimentiert. Nach dem Dekantieren des die lysierten roten Blutzellen enthaltenden Überstands wird das Sediment in 50 ml phosphatgepufferter Salzlösung (PBS) resuspendiert und erneut für 7 Minuten bei 700 x g zentrifugiert. Danach wird das Sediment in 300 µl Ham's F12-Medium ohne Phenolrot mit 1 mM L-Glutamin, 100 Units/ml Penicillin, 100 Units/ml Streptomycin, 50 pg/ml Gentamicin und 1 pg/ml Fungizone (= Amphotericin B), pH 7,4 aufgenommen.

Zur Abgrenzung der Tumorzellen von den übrigen Blutzellen werden 30 µl des bereits gebrauchsfertig vorverdünnten fluorochrommarkierten Antikörpers anti-CD326-FITC (Miltenyi Biotec GmbH, 51429 Bergisch Gladbach, Deutschland) zu der Zellsuspension zugesetzt und für 15 Minuten bei Raumtemperatur in Dunkelheit inkubiert. Anschließend werden 6 ml Ham's F12-Medium mit oben beschriebener Zusammensetzung zugesetzt und die Zellen darin suspensiert. In die Vertiefungen einer Mikrotiterplatte werden je 100 µl PBS für eine Kontrollprobe oder 100 µl PBS mit dem zu testenden Therapeutikum 5-Fluoruracil (Medac Gesellschaft für klinische Spezialpräparate mbH, Theaterstr. 6, 22880 Wedel, Deutschland) in Konzentrationen von 5 ng/ml, 50 ng/ml und 500 ng/ml vorgelegt. Als Indikator für tote Zellen werden zusätzlich jeweils 10 µl einer 5 µg/ml Propidiumiodid-Lösung zugesetzt. Anschließend werden jeweils 100 µl der Zellsuspension zugesetzt.

Zu Beginn der Inkubation und nach 1 Stunde, 3 Stunden, 24 Stunden, 48 Stunden und 72 Stunden wird die in jeder Vertiefung enthaltene Zellsuspension zytometrisch analysiert. Dabei wird jeweils die Anzahl der in einem bestimmten Volumen insgesamt enthaltenen Tumorzellen und der Anteil der absterbenden und toten, d. h. durch Propidiumiodid intrazellulär gefärbten, Tumorzellen an den insgesamt enthaltenen Tumorzellen ermittelt. Anschließend wird von dem für jede der Proben ermittelten Anteil der toten Zellen der für die Kontrollprobe ermittelte Anteil der toten Zellen abgezogen und dadurch die auf das Therapeutikum zurückzuführende zeit- und konzentrationsabhängige Absterberate der Tumorzellen ermittelt.

Fig. 1 zeigt typische Kurvenverläufe für unterschiedliche Konzentrationen des Zytostatikums 5-Fluoruracil nach unterschiedlichen Inkubationszeiten.

## Patentansprüche

1. Verfahren zum Vorhersagen des Ansprechens einer von einem soliden epithelialen Tumor verursachten Tumorerkrankung eines Patienten auf eine therapeutische Maßnahme, wobei epitheliale Tumorzellen aus einer Körperflüssigkeit des Patienten jeweils in einem Zellkulturmedium aufgenommen werden, wobei die Tumorzellen aus einer Probe des die Tumorzellen enthaltenden Zellkulturmediums der therapeutischen Maßnahme ausgesetzt werden, während die Tumorzellen aus einer Kontrollprobe des die Tumorzellen enthaltenden Zellkulturmediums unbehandelt bleiben, wobei anschließend für die Probe und die Kontrollprobe jeweils der Anteil absterbender und toter epithelialer Tumorzellen an der Gesamtzahl der epithelialen Tumorzellen und daraus eine durch die therapeutische Maßnahme bedingte Absterberate der epithelialen Tumorzellen als Maß für das Ansprechen bestimmt wird, wobei das Zellkulturmedium keinen zugesetzten Wachstumsfaktor und kein zugesetztes Serum enthält.

2. Verfahren nach Anspruch 1, wobei zwischen der therapeutischen Maßnahme und dem Bestimmen des Anteils absterbender und toter epithelialer Tumorzellen an der Gesamtzahl der epithelialen Tumorzellen eine Zeit von 1 Stunde bis zu 3 Tagen, insbesondere von 2 Stunden bis zu einem Tag, liegt.

3. Verfahren nach Anspruch 2, wobei die Tumorzellen in der Zeit unter Zellkulturbedingungen gehalten werden, welche ohne therapeutische Maßnahme ein Überleben der Tumorzellen im Zellkulturmedium erlauben.

4. Verfahren nach einem der vorhergehenden Ansprüche, wobei die Körperflüssigkeit Blut, Aszitesflüssigkeit, Lymphe, Pleuraexsudat, Liquor oder Urin ist.

5. Verfahren nach einem der vorhergehenden Ansprüche, wobei die epithelialen Tumorzellen zirkulierende epitheliale Tumorzellen sind (CETCs).

6. Verfahren nach einem der vorhergehenden Ansprüche, wobei die Tumorzellen vor der Aufnahme in das Kulturmedium unter Ausnutzung ihres gegenüber der sie umgebenden Körperflüssigkeit höheren spezifischen Gewichts, insbesondere durch Absetzenlassen oder Zentrifugieren, angereichert werden.

7. Verfahren nach Anspruch 6, wobei die Körperflüssigkeit Blut ist und die darin enthaltenen roten Blutkörperchen vor dem Anreichern der Tumorzellen lysiert werden.

8. Verfahren nach einem der vorhergehenden Ansprüche, wobei die epithelialen Tumorzellen zur Unterscheidung von sonstigen in der Körperflüssigkeit vorhandenen Zellen mit einer spezifisch die Tumorzellen markierenden Substanz, insbesondere einem für epitheliale Zellen spezifischen Antikörper, in Kontakt gebracht werden.

9. Verfahren nach einem der vorhergehenden Ansprüche, wobei den epithelialen Tumorzellen ein spezifisch absterbende und/oder tote Zellen anzeigender erster Indikator, insbesondere Propidiumiodid, oder ein spezifisch lebende Zellen anzeigender zweiter Indikator zugesetzt wird.

10. Verfahren nach einem der vorhergehenden Ansprüche, wobei die therapeutische Maßnahme eine Exposition gegenüber Strahlung oder Wärme oder ein Inkontaktbringen mit einem Zytostatikum, insbesondere 5-Fluoruracil, oder einem sonstigen gegen den Tumor gerichteten Therapeutikum umfasst.

11. Verfahren nach einem der vorhergehenden Ansprüche, wobei der Tumor ein Mammakarzinom oder ein Bronchialkarzinom ist.

## Claims

1. A method for predicting the response of a tumor disease in a patient, caused by a solid epithelial tumor, to a therapeutic measure, wherein epithelial tumor cells from a body fluid of the patient are taken up in a cell culture medium in each case, wherein the tumor cells from a sample of the cell culture medium comprising the tumor cells are exposed to the therapeutic measure, while the tumor cells from a control sample of the cell culture medium comprising the tumor cells remain untreated, wherein the proportion of dying-off and dead epithelial tumor cells in the total number of epithelial tumor cells is then determined for the sample and the control sample, respectively, and is used to determine a dying-off rate of the epithelial tumor cells which is caused by the therapeutic measure as a measure of the response, wherein the cell culture medium does not comprise any added growth factor or added serum.

2. The method as claimed in claim 1, wherein a time interval from 1 hour up to 3 days, in particular from 2 hours up to 1 day, elapses between the therapeutic measure and the determination of the proportion of dying-off and dead epithelial tumor cells in the total number of epithelial tumor cells.

3. The method as claimed in claim 2, wherein during this time interval the tumor cells are kept under cell culture conditions which permit a survival of the tumor cells in the cell culture medium without therapeutic measure.

4. The method according to any of the preceding claims, wherein the body fluid is blood, ascites fluid, lymph, pleural exudate, liquor or urine.

5. The method as claimed in any of the preceding claims, wherein the epithelial tumor cells are circulating epithelial tumor cells (CETCs).

6. The method as claimed in any of the preceding claims, wherein the tumor cells, before being taken up into the culture medium, are enriched exploiting the fact that their specific weight is higher than that of the body fluid surrounding them, in particular by allowing them to settle or by centrifugation.

7. The method as claimed in claim 6, wherein the body fluid is blood and the red blood cells present therein are lyzed before the tumor cells are enriched.

8. The method as claimed in any of the preceding claims, wherein the epithelial tumor cells are brought into contact with a substance which specifically labels the tumor cells, in particular with an antibody with specificity for epithelial cells, so as to distinguish said epithelial tumor cells from other cells which are present in the body fluid.

9. The method as claimed in any of the preceding claims, wherein a first indicator which specifically indicates dying-off and/or dead cells, in particular propidium iodide, or a second indicator which specifically indicates live cells, is added to the epithelial tumor cells.

10. The method as claimed in any of the preceding claims, wherein the therapeutic measure comprises an exposure to radiation or heat or a bringing into contact with a cytostatic, in particular 5-fluorouracil, or with any other therapeutic agent which is directed against the tumor.

11. The method as claimed in any of the preceding claims, wherein the tumor is a mammary carcinoma or a bronchial carcinoma.

## Revendications

1. Procédé de prédiction de la réponse d'une maladie tumorale provoquée par une tumeur épithéliale solide d'un patient à une mesure thérapeutique, dans lequel des cellules tumorales épithéliales provenant d'un fluide corporel du patient sont à chaque fois incorporées dans un milieu de culture cellulaire, dans lequel les cellules tumorales provenant d'un échantillon du milieu de culture cellulaire contenant les cellules tumorales sont exposées à la mesure thérapeutique tandis que les cellules tumorales provenant d'un échantillon témoin du milieu de culture cellulaire contenant les cellules tumorales restent non traitées, dans lequel la proportion de cellules tumorales épithéliales nécrosantes et mortes sur le nombre total des cellules tumorales épithéliales est ensuite à chaque fois déterminée pour l'échantillon et l'échantillon témoin et un taux de nécrose des cellules tumorales épithéliales conditionné par la mesure thérapeutique est déterminé à partir de celle-ci en tant que mesure pour la réponse, dans lequel le milieu de culture cellulaire ne contient aucun facteur de croissance ajouté et aucun sérum ajouté.

2. Procédé selon la revendication 1, dans lequel une durée de 1 heure à 3 jours, notamment de 2 heures à un jour, se situe entre la mesure thérapeutique et la détermination de la proportion de cellules tumorales épithéliales nécrosantes et mortes sur le nombre total des cellules tumorales épithéliales.

3. Procédé selon la revendication 2, dans lequel les cellules tumorales sont maintenues pendant la durée dans des conditions de culture cellulaire qui permettent sans mesure thérapeutique une survie des cellules tumorales dans le milieu de culture cellulaire.

4. Procédé selon une des revendications précédentes, dans lequel le fluide corporel est du sang, du liquide ascitique, de la lymphe, de l'exsudat pleural, du liquide céphalorachidien ou de l'urine.

5. Procédé selon une des revendications précédentes, dans lequel les cellules tumorales épithéliales sont des cellules tumorales épithéliales circulantes (CETC).

6. Procédé selon une des revendications précédentes, dans lequel les cellules tumorales sont enrichies avant l'incorporation dans le milieu de culture en exploitant leur poids spécifique supérieur par rapport au fluide corporel les entourant, notamment par précipitation ou centrifugation.

7. Procédé selon la revendication 6, dans lequel le fluide corporel est du sang et les globules rouges contenus dans celui-ci sont lysés avant l'enrichissement des cellules tumorales.

8. Procédé selon une des revendications précédentes, dans lequel les cellules tumorales épithéliales sont amenées en contact avec une substance marquant spécifiquement les cellules tumorales, notamment un anticorps spécifique pour des cellules épithéliales, pour la différenciation d'autres cellules présentes dans le fluide corporel.

9. Procédé selon une des revendications précédentes, dans lequel un premier indicateur indiquant spécifiquement des cellules nécrosantes et/ou mortes, notamment de l'iodure de propidium, ou un second indicateur indiquant spécifiquement des cellules vivantes est ajouté aux cellules tumorales épithéliales.

10. Procédé selon une des revendications précédentes, dans lequel la mesure thérapeutique comprend une exposition à un rayonnement ou de la chaleur ou une mise en contact avec un agent cytostatique, notamment du 5-fluorouracile, ou un autre agent thérapeutique dirigé contre la tumeur.

11. Procédé selon une des revendications précédentes, dans lequel la tumeur est un carcinome mammaire ou un carcinome bronchique.
